# EUROPEAN PATENT APPLICATION

(11) **EP 2 586 407 A1**
(43) Date of publication of application: **01.05.2013**
(21) Application number: 11187240.4
(22) Date of filing: 31.10.2011
(51) Int. Cl.: A61F 9/00, A61F 9/013

(54) **Aid device for intraocular injection**

(71) Applicant: Sanofi-Aventis Deutschland GmbH, 65929 Frankfurt am Main (DE)
(72) Inventor: The designation of the inventor has not yet been filed

(57) **Abstract**

Described is an aid device for an intraocular injection comprising a skirt (1) having an aperture to receive an eye, and a cover (2) coupled to the skirt (1). The cover (2) has one or more guide holes (4) adapted to receive a needle.

## Description

### Background

An intraocular injection device may be used to administer therapeutic sub-stances to eyes, such as eyes of mammals having eye disorders or diseases.

A number of vision-threatening disorders or diseases of the eye need to deliver a medicament (pharmaceutical, biological, etc.) and/or implantable device to a posterior segment of the eye by intraocular delivery (more specifically intravitreal delivery). One such technique for intraocular delivery is accomplished by intraocular injection into the vitreous body. A conventional apparatus for intraocular injection may include a pre-filled syringe of a medicament.

When a treatment protocol calls for a series of injections, previous injection sites may not be taken into account when administering subsequent injections. That is, injections may be delivered to the same injection site on more than one occasion, which can inhibit the healing process.

Additionally, intraocular injections must take into account surrounding ocular structures which should be avoided during the injection. Great manual dexterity is required to administer such injections.

Therefore, there is a need for an aid device for use when administering intraocular injections which facilitates guiding the injection device and promotes healing of an injection site.

### Summary of the Invention

It is an object of the present invention to provide for an apparatus that aids in guiding an intraocular injection device and administering an intraocular injection.

In an exemplary embodiment, an aid device for an intraocular injection according to the present invention comprises a skirt having an aperture to receive an eye, and a cover coupled to the skirt. The cover has one or more guide holes adapted to receive a needle.

In an exemplary embodiment, the skirt includes a rim adapted to abut eyelids.

In an exemplary embodiment, the cover includes one or more targets.

In an exemplary embodiment, the cover includes one or more alignment guides.

In an exemplary embodiment, the aid device further comprises a port adapted to provide access to an interior of the skirt.

In an exemplary embodiment, the aid device further comprises a reservoir adapted to contain a therapeutic substance.

In an exemplary embodiment, the aid device further comprises a conjunctiva displacement device having a first end adapted to contact a surface of the eye and a second end extending proximally of the cover. The first end is an anchor.

The person skilled in the art understands that the present invention is not re-stricted to the explained possibilities.

The above mentioned advantages as well as other advantages of various as-pects of the present invention will become apparent to those of ordinary skill in the art by reading the following detailed description, with appropriate reference to the accompanying drawings.

### Brief Description of the Drawings

In the following, the invention will be described by way of an example and with reference to the schematic drawings in which:
Figure 1 shows a top view of an aid device according to an exemplary embodiment of the present invention,
Figure 2 shows a side view of an exemplary embodiment of an aid device according to the present invention,
Figure 3 shows a section of an exemplary embodiment of an aid device according to the present invention, and
Figure 4 shows a section of a manipulator according to another exemplary embodiment of an aid device according to the present invention.

### Detailed Description

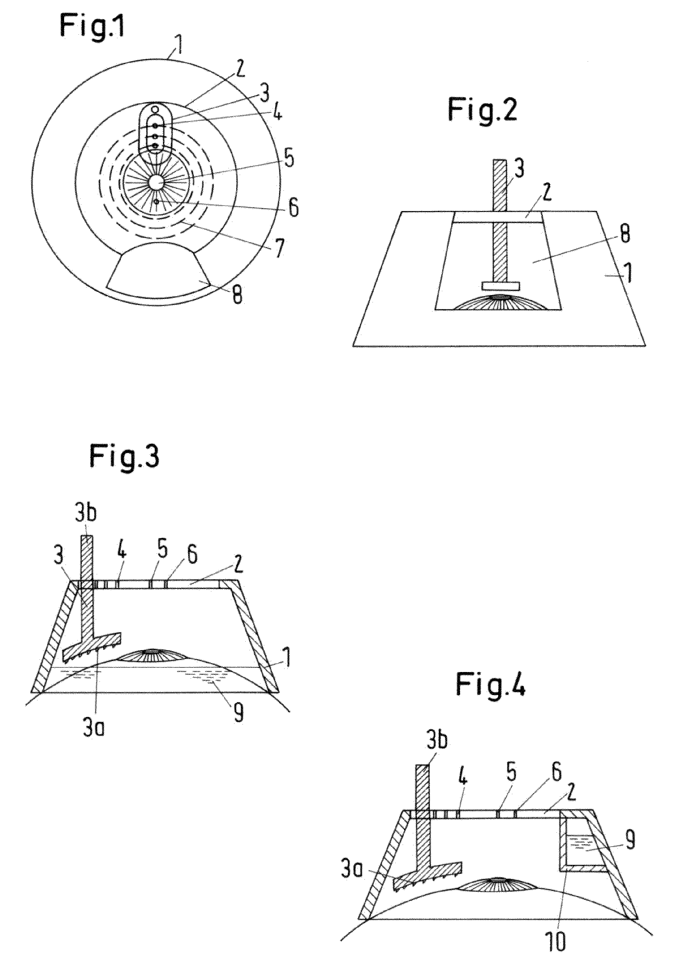

Figures 1 to 3 show an exemplary embodiment of an aid device for an intraocular injection. The device comprises a skirt 1 which is adapted to be positioned on a surface of an eye. A distal portion of the skirt 1 may include an aperture for receiving the eye, and a proximal portion of the skirt 1 may include a cover 2. The aperture of the skirt 1 may have a rim with a circular, oval or elliptical cross-section to facilitate placement on the eye. Further, the rim may be adapted to abut opposing eyelids of the eye to maintain the eyelids in a separated state while the device is on the eye. As understood by those of skill in the art, the aid device may be manufactured from a translucent or transparent material (which may include a magnification property) to define a visual field around an injection site.

In an exemplary embodiment, the device may include or be utilized with a conjunctiva displacement device 3. The conjunctiva displacement device 3 may have a first end 3a which is adapted to engage a conjunctiva on the eye and a second end 3b which extends proximally of the cover 2. When the second end 3b is moved, the first end 3a may move in conjunction and displace the conjunctiva relative to a sclera. By displacing the conjunctiva relative to the sclera prior to an injection, injection sites in these layers will be axially offset when the layers return to their respective pre-injection positions. Covering the injection site in the sclera with a solid conjunctiva may prevent reflux of an injected medicament and may promote healing of both injection sites. In another exemplary embodiment, the conjunctiva displacement device 3 may be an anchor which maintains the conjunctiva in place relative to the sclera as the eye rotates.

In an exemplary embodiment, the cover 2 is provided one or more guide holes 4 adapted to receive a needle. More than one guide hole 4 may be utilized when the aid device is used for a series of injections and it is desired that consecutive injections not be administered in or adjacent to the same injection site. Further, the guide holes 4 may be positioned offset from a central axis of the aid device to prevent the needle from piercing an unintended anatomical feature during the injection.

In an exemplary embodiment, one or more targets 5, 6 may be provided on the cover 2. The targets 5, 6 may have the form of colored dots, protrusions, re-cesses, openings or the like. The targets 5, 6 may be utilized with the conjunctiva displacement device 3 and/or the conjunctiva anchor, by having a patient look at the various targets 5, 6 to cause displacement of the sclera relative to the conjunctiva, or vice-versa, prior to the injection.

In an exemplary embodiment, one or more alignment guides 7 may be provided on the cover 2. In the exemplary embodiment depicted in Figure 1, the alignment guides 7 have the form of concentric circles which are spaced from each other by approximately 4 mm. A given hole 4 may be positioned on each of the alignment guides 7. The alignment guides 7 may have the form of a dashed or dotted lines or may have the form of a continuous line. The alignment guides 7 circles may be made as colored lines, circular protrusions, circular grooves or the like. In an exemplary embodiment, the alignment guides 7 are arranged on the cover 2 such that they are concentric with the center of window 2.

In an exemplary embodiment, a port 8 is provided in the aid device. The port 8 may provide access to an interior of the aid device or a reservoir 10 (shown in Figure 4). The port 8 may be utilized for disposing a therapeutic substance 9, e.g., an anesthetic, disinfectant, antimicrobial, etc., on the eye before, during or after an injection or providing suction to the injection site. The reservoir 10 may be opened to allow, e.g., an anesthetic, disinfectant, antimicrobial, etc., on the eye.

In an exemplary use of an exemplary embodiment of the aid device, the aid device is placed on the eye, and the skirt 1 maintains the eyelids in a separated state. The skirt 1 may be adapted to sealingly engage the eye such that a volume of anesthetic and disinfectant may be applied via the port 8 and/or the reservoir 10 before, during or after the injection.

The patient is then requested to look a first target 6 on the cover 2. The conjunctiva displacement device 3 is then lowered to grip the conjunctiva, before the patient is told to look at the second target 5 in the window 2 directly above the patient's cornea. The act of looking at the second target 5 moves the conjunctiva relative to the sclera, or vice-versa, and sets the position of the eye.

A guide hole 4 is selected for the injection and the needle is inserted into the eye via the selected guide hole 4. When the injection is complete, the needle is removed and the conjunctiva displacement device 3 is lifted to allow the conjunctiva and/or sclera to return to their respective initial positions. A volume of anesthetic and disinfectant may be applied via the port 8 and/or the reservoir 10 after the injection.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

## Claims

1. An aid device for an intraocular injection, comprising:
a skirt (1) having an aperture to receive an eye;
a cover (2) coupled to the skirt (1), the cover (2) having one or more guide holes (4) adapted to receive a needle.

2. The aid device according to claim 1, wherein the skirt (1) includes a rim adapted to abut eyelids.

3. The aid device according to any of the preceding claims, wherein the cover (2) includes one or more targets (5, 6).

4. The aid device according to any of the preceding claims, wherein the cover (2) includes one or more alignment guides (7).

5. The aid device according to any of the preceding claims, further comprising:
a port (8) adapted to provide access to an interior of the skirt (1).

6. The aid device according to any of the preceding claims, further comprising:
a reservoir (10) adapted to contain a therapeutic substance (9).

7. The aid device according to any of the preceding claims, further comprising:
a conjunctiva displacement device (3) having a first end (3a) adapted to contact a surface of the eye and a second end (3b) extending proximally of the cover (2).

8. The aid device according to claim 7, wherein the first end (3a) is an anchor.
